# EUROPÄISCHE PATENTSCHRIFT

(11) **EP 1 984 740 B1**
(45) Veröffentlichungstag und Bekanntmachung des Hinweises auf die Patenterteilung: **08.02.2012**
(21) Anmeldenummer: 06818485.2
(22) Anmeldetag: 10.11.2006
(51) Int. Cl.: G01N 33/569, B01L 7/00, A61J 1/00, F17C 3/02

(54) **PROBENSAMMELVERFAHREN**
SAMPLE COLLECTION METHOD
PROCÉDÉ DE PRÉLÈVEMENT D'ÉCHANTILLONS

(30) Priorität: 16.02.2006 DE 102006007315
(43) Veröffentlichungstag der Anmeldung: 29.10.2008
(73) Patentinhaber: Fraunhofer-Gesellschaft zur Förderung der angewandten Forschung e.V., 80686 München (DE)
(72) Erfinder: ZIMMERMANN, Heiko, 66386 St. Ingbert (DE); VON BRIESEN, Hagen, 65510 Hünstetten (DE); FUHR, Günter, 13187 Berlin (DE)
(74) Vertreter: Beier, Ralph
(86) Internationale Anmeldenummer: PCT/EP2006/010826
(87) Internationale Veröffentlichungsnummer: WO 2007/093205

(56) Entgegenhaltungen:
- WO-A-01/18239
- WO-A-03/073992
- WO-A-2004/048615
- WO-A-2005/019835
- WO-A-2005/113147
- WO-A-2005/115621
- GB-A- 824 702
- US-A- 3 092 974
- US-A- 4 790 141
- US-A1- 2003 183 683
- US-A1- 2005 178 218
- FERNANDO LEONOR P ET AL: "Would nucleic acid testing (NAT) have prevented HIV transmission from window period platelet donations?" BLOOD, Bd. 96, Nr. 11 Part 1, 16. November 2000 (2000-11-16), Seite 59a, XP008079631 & 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- MALHOTRA RAJESH ET AL: "P24 ANTIGEN SCREENING TO REDUCE THE RISK OF HIV TRANSMISSION BY SERONEGATIVE BONE ALLOGRAFT DONORS" NATIONAL MEDICAL JOURNAL OF INDIA, OXFORD UNIVERSITY PRESS, NEW DEHLI, IN, Bd. 13, Nr. 4, Juli 2000 (2000-07), Seiten 190-192, XP008079636 ISSN: 0970-258X
- BUSCH MICHAEL P ET AL: "TIME COURSE OF VIREMIA AND ANTIBODY SEROCONVERSION FOLLOWING HUMAN IMMUNODEFICIENCY VIRUS EXPOSURE" AMERICAN JOURNAL OF MEDICINE, XX, XX, Bd. 102, Nr. 5 PART B, 1997, Seiten 117-124, XP008079615 ISSN: 0002-9343
- KOPKO PM ET AL: "HIV Tansmission From a Window-Period Platelet Donation", AM J CLIN PATHOL, vol. 116, 2001, pages 562-566,
- ANONYMOUS: 'Seroconversion', [Online] 15 Dezember 2005, XP007918142 Gefunden im Internet: <URL:http://replay.web.archive.org/20051215 000000/http://en.wikipedia.org/wiki/Serocon version> [gefunden am 2011-05-11]

## Beschreibung

Die Erfindung betrifft ein Probensammelverfahren zur Gewinnung von Blutproben von frisch HIV-Infizierten während der diagnostischen Lücke eines Antikörper-basierten HIV-Tests.

In der Aids-Forschung sind sogenannte "Early-Infection"-Blutproben von HIV-Infizierten von besonderem wissenschaftlichem Interesse. Dabei handelt es sich um Blutproben von HIV-Infizierten, deren Infektion erst vor sehr kurzer Zeit erfolgt ist. Bisher wurden zur Bereitstellung von Blutproben von HIV-Infizierten Antikörper-basierte Tests durchgeführt, wobei den getesteten Personen bei einem positiven Testergebnis Blutproben für eine wissenschaftliche Untersuchung abgenommen wurden.

Nachteilig an diesem bekannten Verfahren zur Bereitstellung von Blutproben von HIV-Infizierten ist die zeitliche diagnostische Lücke der verwendeten Antikörper-basierten HIV-Tests, die beispielsweise zwölf Wochen betragen kann. Bei den positiv getesteten Probanden liegt zwischen der HIV-Infektion und dem positiven Test also zumindest die diagnostische Lücke des verwendeten HIV-Tests, so dass die auf diese Weise bereitgestellten Proben keine "Early-Infection"-Proben sind.

Ein Testverfahren zum Ausschluss von HIV-verseuchten Proben während des diagnostischen Fensters ist auch bekannt aus Fernando Leonor P. et al.: "Would nucleid acid testing (NAT) have prevented HIV transmission from window period platelet donations?" BLOOD, Bd. 96, Nr. 11 Part 1, 16. November 2000 (2000-11-16) Seite 59a, XP008079631 & 42ND Annual meeting of the American society of haematology; San Francisco, California, USA; December 01-05, 2000 ISSN: 0006-4971. Aus dieser Veröffentlichung ist jedoch nicht bekannt, gezielt sogenannte "Early-Infection"-Proben für eine wissenschaftliche Verwertung zu sammeln.

Ferner ist zum Stand der Technik noch hinzuweisen auf Malhotra Fajesh et al: "P24 antigen screening to reduce the risk of HIV transmission by seronegative bone allograft donors", National medical journal of India, Oxford university press, New Dehli, Bd. 13, Nr. 4, Juli 2000, Seiten 190-192, ISSN: 0970-258X, Busch, Michael et al.: "Time course of viremia and antibody seroconversion following human immunodeficiency virus exposure", American journal of medicine, Bd. 102, Nr. 5, Part B, 1997, Seiten 117-124, ISSN: 0002-9343, WO 2004/048615 A, WO 03/073992 A, GB 824 702 A, US-A-3 092 974, US-A-4 790 141, WO 2005/113147 A, WO 2005/019835 A, WO 2005/115621 A, US 2003/183683 A1, US 2005/178218.A1 und WO 01/18239 A.

Der Erfindung liegt deshalb die Aufgabe zugrunde, "Early-Infection"-Proben von HIV-Infizierten zum frühestmöglichen Zeitpunkt nach der HIV-Infektion bereitzustellen.

Diese Aufgabe wird durch ein Probensammelverfahren gemäß dem unabhängigen Anspruch gelöst.

Im Rahmen des erfindungsgemäßen Probensammelverfahrens erfolgt zunächst eine erste Probenentnahme von biologischen Proben von einer Vielzahl von Lebewesen einer Stichprobe.

Anschließend werden die entnommenen Proben mit einem ersten Diagnosetest auf einen bestimmten interessierenden Stoff hin untersucht, wobei es sich um das HIV-Virus handelt Diese erste Untersuchung der entnommenen Proben hat den Zweck, diejenigen untersuchten Lebewesen aus der Stichprobe auszuschließen, die bereits seit längerem von dem interessierenden Stoff (z.B. dem HIV-Virus) befallen sind und deshalb keine "Early-Infection"=Proben liefern können. Diejenigen Lebewesen, die zu den positiv getesteten Proben gehören, werden deshalb aus der Stichprobe ausgeschlossen, da nur "Early-Infection"-Proben gesucht werden.

Die negativ-getesteten Proben werden dagegen konserviert, da es sich bei diesen Proben potentiell um "Early-Infection"-Proben handeln kann, die nur deshalb nicht zu einem positiven Testergebnis geführt haben, da der Befall mit dem interessierenden Stoff (z.B. dem HIV-Virus) innerhalb der diagnostischen Lücke des verwendeten Diagnosetest erfolgt ist.

Nach der ersten Probenentnahme wird dann für eine bestimmte Wartezeit abgewartet, um anschließend weitere Untersuchungen zu ermöglichen.

Nach Ablauf der Wartezeit erfolgt dann eine zweite Probenentnahme von biologischen Proben von den in der Stichprobe verbliebenen Lebewesen und eine zweite Untersuchung der entnommenen Proben mit einem zweiten Diagnosetest.

Schließlich werden dann die konservierten Proben derjenigen Lebewesen selektiert, die bei dem ersten Diagnosetest negativ rund bei dem zweiten Diagnosetest positiv getestet wurden. Das negative Testergebnis bei den ersten Diagnosetests schließt hierbei die bereits seit längerem von dem interessierenden Stoff befallenen Lebewesen aus, während das positive Testergebnis bei dem zweiten Diagnosetest diejenigen Proben auswählt, die bei der ersten Probenentnahme bereits von dem interessierenden Stoff befallen waren, obwohl dieser Befall aufgrund der diagnostischen Lücke des ersten Diagnosetests nicht zu einem positiven Testergebnis geführt hat. Bei den auf diese Weise selektierten Proben handelt es sich deshalb mit sehr großer Wahrscheinlichkeit um die gesuchten "Early-Infection"-Proben.

Bei den entnommenen Proben handelt sich um Blutproben.

Ferner ist die Erfindung hinsichtlich der zu beprobenden Lebewesen nicht auf Menschen beschränkt, sondern grundsätzlich auch bei Tieren anwendbar.

Die Konservierung der entnommenen Proben zwischen den einzelnen Probenentnahmen erfolgt durch eine Kryokonservierung, wobei die Kryokonservierung vorzugsweise vitalitätserhaltend erfolgt, was an sich bekannt ist.

Weiterhin ist zu erwähnen, dass die Wartezeit zwischen der ersten Probenentnahme und der zweiten Probenentnahme kürzer sein sollte als die zeitliche diagnostische Lücke des zweiten Diagnosetests. Auf diese Weise wird die Wahrscheinlichkeit dafür erhöht, dass ein positives Testergebnis bei der zweiten Probenentnahme auf einer Infektion beruht, die bereits vor der ersten Probenentnahme erfolgt ist, so dass die bei der ersten Probenentnahme entnommenen Proben bereits von dem interessierenden Stoff befallen sind. Falls dagegen ein Befall mit dem interessierenden Stoff in dem Zeitintervall zwischen der ersten Probenentnahme und der zweiten Probenentnahme erfolgt ist, führt der zweite Diagnosetest bei der zweiten Probenentnahme aufgrund der diagnostischen Lücke in der Regel nicht zu einem positiven Testergebnis, da die diagnostische Lücke zu diesem Zeitpunkt noch nicht abgelaufen ist.

Es ist jedoch zu erwähnen, dass die diagnostische Lücke lediglich eine statistische Kenngröße des verwendeten Diagnosetests ist. Es besteht deshalb eine statistische Möglichkeit, dass der zweite Diagnosetest bei der zweiten Probenentnahme auch dann positiv anspricht, wenn die diagnostische Lücke seit der vorangegangenen Infektion noch nicht abgelaufen ist. Beispielsweise kann auch eine HIV-Infektion in dem Zeitintervall zwischen der ersten Probenentnahme und der zweiten Probenentnahme aufgrund statistischer Schwankungen der diagnostischen Lücke zu einem positiven Testergebnis bei der zweiten Probenentnahme führen. Dies hätte die Folge, dass die bei der ersten Probenentnahme entnommenen Proben fälschlicherweise als "Early-Infection"-Proben klassifiziert würden. Die Wahrscheinlichkeit derartiger Fehlklassifikationen von Proben als "Early-Infection"-Proben lässt sich durch eine Verkürzung der Wartezeit zwischen der ersten Probeentnahme und der zweiten Probenentnahme verringern. Andererseits führt eine Verkürzung der Wartezeit zwischen der ersten Probenentnahme und der zweiten Probenentnahme zu einer Verringerung der Ausbeute von "Early-Infection"-Proben, d.h. tatsächliche "Early-Infection"-Proben werden fälschlicherweise nicht als solche erkannt. Zur Optimierung dieser gegenläufigen Ziele lässt sich die Wartezeit zwischen der ersten Probenentnahme und der zweiten Probenentnahme entsprechend einstellen, wobei die Wartezeit beispielsweise 10-90%, 20-80%, 30-70% oder 40-60% der diagnostischen Lücke des zweiten Diagnosetests betragen kann.

Zur Vermeidung der Fehlklassifikation von Proben als "Early-Infection"-Proben wird jedoch zusätzlich eine dritte Untersuchung der Proben mit einem dritten Diagnosetest erfolgen, wobei der dritte Diagnosetest eine kürzere diagnostische Lücke aufweist als der erste Diagnosetest und/oder der zweite Diagnosetest. Beispielsweise kann es sich bei dem dritten Diagnosetest um einen Antigen-Test, insbesondere einen HIV-Antigen-Test, handeln, der eine kürzere diagnostische Lücke aufweist als die kostengünstigeren Antikörper-basierten Virustests, wie beispielsweise der bekannte HIV-Elisa-Test. Durch den dritten Diagnosetest können dann diejenigen Proben ausgeschlossen werden, die aufgrund der statistischen Schwankung der diagnostischen Lücke des zweiten Diagnosetest fälschlicherweise als "Early-Infection"-Proben erkannt wurden, obwohl die Infektion tatsächlich nach der jeweiligen Probenentnahme erfolgt ist. Beispielsweise kann bei der dritten Untersuchung ein RT-PCR-Test durchgeführt werden.

Die kryokonservierten Proben werden nach der Selektion der mit dem interessierenden Stoff befallenen Proben aufgetaut, um eine weitere wissenschaftliche Untersuchung zu ermöglichen. Es besteht jedoch im Rahmen der Erfindung auch die Möglichkeit, dass die selektierten Proben lediglich als "Early-Infection"-Proben markiert werden, um eine spätere Auswertung zu erlauben. Die Markierung der "Early-Infection"-Proben erfolgt vorzugsweise durch eine Identifikationsmarkierung, die an dem jeweiligen Probenbehälter angebracht wird, wobei es sich beispielsweise um einen optischen Code, insbesondere einen Strichcode, einen RFID-Transponder oder einen Mikrochip handeln kann. Die Identifikationsmarkierung der einzelnen Probenbehälter mit den darin befindlichen "Early-Infection"-Proben ist aus logistischen Gründen insbesondere dann wichtig, wenn eine Vielzahl von Lebewesen beprobt werden soll.

Im Rahmen des erfindungsgemäßen Probensammelverfahrens werden die bei dem ersten und/oder zweiten Diagnosetest positiv getesteten Proben vorzugsweise vernichtet, da es sich bei diesen Proben aufgrund der unvermeidbaren diagnostischen Lücke der jeweiligen Diagnosetests nicht um "Early-Infection"-Proben handelt.

Dies bezieht sich jedoch nur auf die beiden ersten Diagnosetests, die eine relativ lange diagnostische Lücke aufweisen. Falls dagegen zur Vermeidung von Fehlklassifikationen von Proben als "Early-Infection"-Proben zusätzlich die vorstehend erwähnte dritte Untersuchung durchgeführt wird, werden vorzugsweise die Proben vernichtet, die bei der dritten Untersuchung negativ getestet wurden und deshalb keine "Early-Infection"-Proben sind.

In einer anderen Variante der Erfindung erfolgt jedoch keine Vernichtung der positiv getesteten Proben, sondern eine korrelierte Auswertung der "Early-Infection"-Proben mit den später entnommenen positiv getesteten Proben.

Wichtig ist ferner, dass die Kryokonservierung der Proben jeweils innerhalb einer vorgegebenen Konservierungsfrist nach der jeweiligen Probenentnahme erfolgt, um eine Veränderung der Proben in der Zwischenzeit zu vermeiden. Die Konservierungsfrist ist deshalb vorzugsweise kürzer als eine Stunde, dreißig Minuten, zehn Minuten oder fünf Minuten.

Weiterhin ist zu erwähnen, dass die Probenentnahme vorzugsweise jeweils dezentral am Aufenthaltsort der zu beprobenden Lebewesen erfolgt, um eine Vielzahl von Lebewesen beproben zu können.

Andere vorteilhafte Weiterbildungen der Erfindung sind in den Unteransprüchen gekennzeichnet oder werden nachstehend zusammen mit der Beschreibung der bevorzugten Ausführungsbeispiele der Erfindung anhand der Figuren näher erläutert. Es zeigen:
- Figuren 1a, 1b: ein Ausführungsbeispiel eines erfindungsgemäßen Probensammelverfahrens zur Gewinnung von "Early-Infection"-Blutproben von HIV-Infizierten in Form eines Flussdiagramms,
- Figur 2a-2c: verschiedene Zeitdiagramme zur Verdeutlichung des Probensammelverfahrens gemäß den Figuren 1a und 1b,
- Figur 3: eine grob vereinfachte Darstellung einer Probensammeleinrichtung sowie
- Figuren 4a-4c: ein alternatives Ausführungsbeispiel eines erfindungsgemäßen Probensammelverfahrens, bei dem mehrere Beprobungszyklen zeitlich aufeinander folgen.

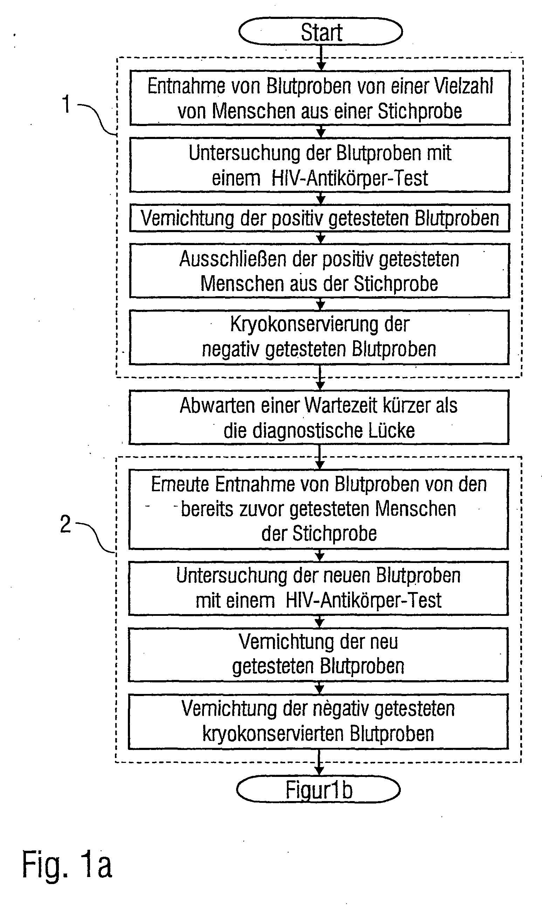

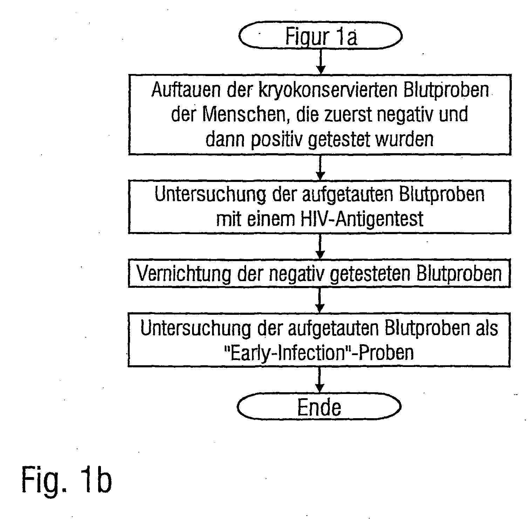

Das in den Figuren 1 und 1a und 1b in Form eines Flussdiagramms dargestellte erfindungsgemäße Probensammelverfahren dient zur Bereitstellung von "Early-Infection"-Blutproben von frisch HIV-Infizierten, damit die "Early-Infection"-Blutproben anschließend wissenschaftlich untersucht werden können.

Das Probensammelverfahren besteht im Wesentlichen aus zwei Abschnitten 1, 2, die in Figur 1a dargestellt sind, und einem weiteren Abschnitt, der in Figur 1b dargestellt ist.

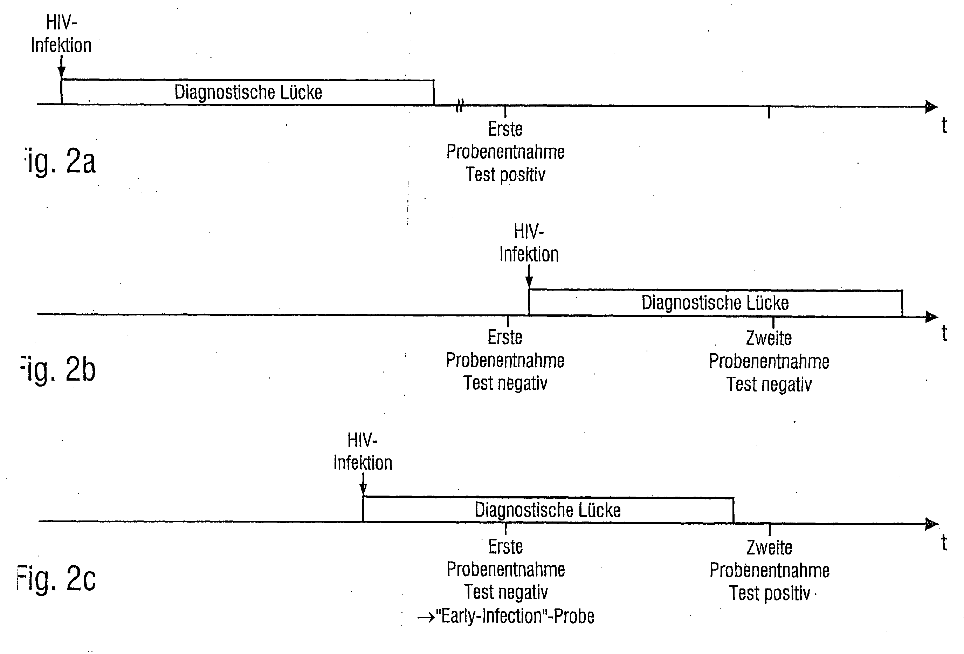

In dem ersten Abschnitt 1 sollen zunächst diejenigen Blutproben ausgeschlossen werden, die von Probanden stammen, die bereits seit längerem HIV-infiziert sind und deshalb zur Gewinnung von "Early-Infection"-Blutproben ungeeignet sind, wie in Figur 2a dargestellt ist.

In dem Abschnitt 1 des erfindungsgemäßen Probensammelverfahrens erfolgt deshalb zunächst eine Entnahme von Blutproben von einer Vielzahl von Menschen aus einer vorgegebenen Stichprobe. Die Stichprobe besteht hierbei vorzugsweise aus Hochrisikogruppen, die eine hohe Infektionsrate erwarten lassen, wodurch mit einer hohen Ausbeute von "Early-Infection"-Blutproben zu rechnen ist.

Die entnommenen Blutproben werden dann mit einem HIV-Antikörper-Test auf eine HIV-Infektion hin untersucht, wozu beispielsweise der bekannte HIV-Elisa-Test verwendet werden kann.

Die positiv getesteten Blutproben werden dann vernichtet, da diese Blutproben zumindest bereits seit einem Zeitraum infiziert sind, der größer ist als die diagnostische Lücke des verwendeten HIV-Antikörper-Tests, so dass die positiv getesteten Blutproben keine "Early-Infection"-Blutproben sind.

Darüber hinaus werden diejenigen Menschen, deren Blutproben positiv getestet wurden, aus der Stichprobe ausgeschlossen, da diese Menschen aufgrund ihrer bereits bestehenden HIV-Infektion auch künftig keine "Early-Infection"-Blutproben liefern können.

Die negativ getesteten Blutproben werden dagegen in herkömmlicher Weise kryokonserviert, wodurch ihr Zustand als "Early-Infection"-Blutproben auch über einen längeren Lagerungszeitraum erhalten werden kann. Bei den kryokonservierten Blutproben handelt es sich jedoch nicht nur um "Early-Infection"-Blutproben, sondern auch um nicht-infizierte Blutproben, die somit keine "Early-Infection"-Blutproben sind und deshalb noch ausselektiert werden müssen.

Zum Ausselektieren der nicht-infizierten kryokonservierten Blutproben dient der Abschnitt 2 des erfindungsgemäßen Probensammelverfahrens, wobei zwischen den beiden Abschnitten 1 und 2 eine Wartezeit abgewartet wird, die kürzer ist als die diagnostische Lücke des verwendeten HIV-Antikörper-Tests.

Nach dem Ablauf der Wartezeit erfolgt dann erneut eine Entnahme von Blutproben von den bereits zuvor getesteten und in der Stichprobe verbliebenen Menschen.

Die nun neu entnommenen Proben werden dann wieder mit einem HIV-Antikörper-Test auf eine HIV-Infektion, hin getestet.

Die neu getesteten Blutproben werden dann in diesem Ausführungsbeispiel vernichtet, da diese Blutproben nicht weiter benötigt werden. Es besteht jedoch alternativ die Möglichkeit, auch die neu getesteten Blutproben auszuwerten, was eine korrelierte Auswertung von "Early-Infection"-Blutproben mit den anschließend entnommenen Blutproben ermöglicht.

Darüber hinaus werden auch diejenigen zuvor kryokonservierten Blutproben vernichtet, die nun bei dem zweiten HIV-Antikörper-Test negativ getestet wurden. Bei diesen Blutproben handelt es sich nämlich mit größter Wahrscheinlichkeit um Blutproben, die zum Zeitpunkt der ersten Probenentnahme noch nicht HIV-infiziert waren, was in Figur 2b dargestellt ist.

Die verbliebenen kryokonservierten Proben führten also bei der ersten Probenentnahme zu einem negativen Testergebnis und bei der zweiten Probe zu einem positiven Testergebnis, was in Figur 2c dargestellt ist. Diese kryokonservierten Proben sind also mit sehr großer Wahrscheinlichkeit "Early-Infection"-Blutproben.

Aufgrund der statistischen Schwankung der Zeitdauer der diagnostischen Lücke besteht jedoch auch bei diesen Blutproben die Möglichkeit, dass die HIV-Infektion eigentlich erst in den Zeitintervall zwischen den beiden Probenentnahmen erfolgt ist, wobei das positive Testergebnis bei der zweiten Probenentnahme durch eine statistische Verkürzung der diagnostischen Lücke verursacht wurde. Eine derartige statistische Schwankung der diagnostischen Lücke kann zu einer Fehlklassifikation von nicht-infizierten Blutproben als "Early-Infection"-Blutproben führen.

Zur weiteren Minimierung dieser statistischen Möglichkeit einer Fehlklassifikation von Blutproben werden die kryokonservierten Blutproben in dem Abschnitt in Figur 1b aufgetaut und anschließend mit einem HIV-Antigentest getestet, der eine wesentlich kürzere diagnostische Lücke aufweist als der HIV-Antikörpertest.

Die dabei negativ getesteten Blutproben werden dann vernichtet, da es sich hierbei mit großer Wahrscheinlichkeit um Blutproben handelt, die bei der ersten Probenentnahme noch nicht infiziert waren.

Die auf diese Weise selektierten übrig gebliebenen Blutproben werden dann als "Early-Infection"-Blutproben wissenschaftlich untersucht, um weitere Erkenntnisse im Rahmen der AIDS-Forschung zu gewinnen.

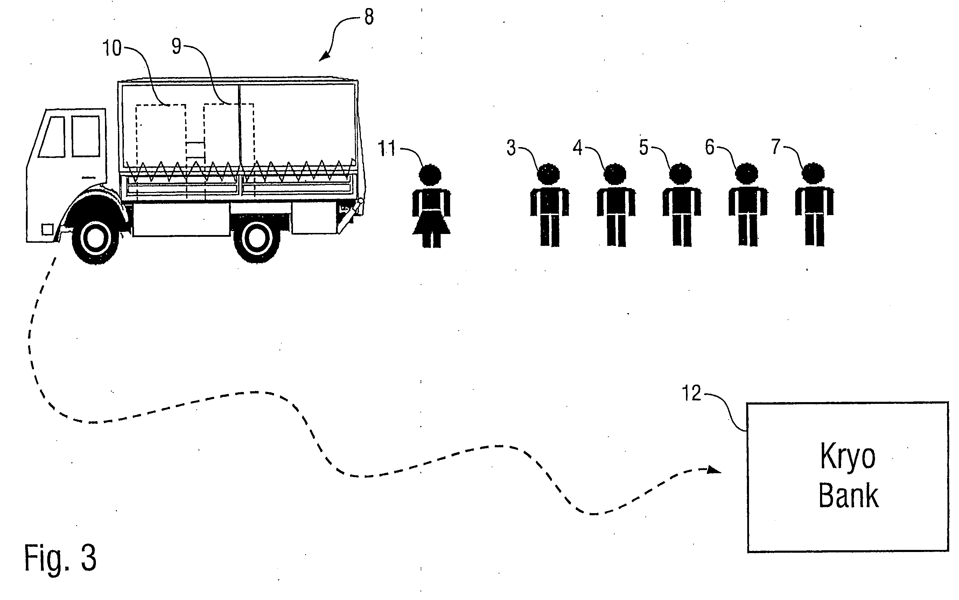

Figur 3 zeigt in grob vereinfachter schematischer Darstellung eine Probensammeleinrichtung, die zur Massenuntersuchung von Menschen 3, 4, 5, 6, 7 zur Gewinnung von "Early-Infection"-Blutproben geeignet ist.

Die Probensammeleinrichtung weist einen Lastkraftwagen 8 auf, auf dessen Ladefläche ein Kryobehälter 9 und ein Einfrierautomat 10 angeordnet sind. Eine Krankenschwester 11 kann den zu untersuchenden Menschen 3-7 dann jeweils eine Blutprobe abnehmen und diese in dem Kryobehälter 9 vitalitätserhaltend zwischenlagern.

Der Lastkraftwagen 8 mit den auf diese Weise dezentral aufgenommenen Blutproben der Menschen 3-7 wird dann zu einer Kryobank 12 gefahren, wo die weitere Auswertung und Lagerung der entnommenen Blutproben erfolgt.

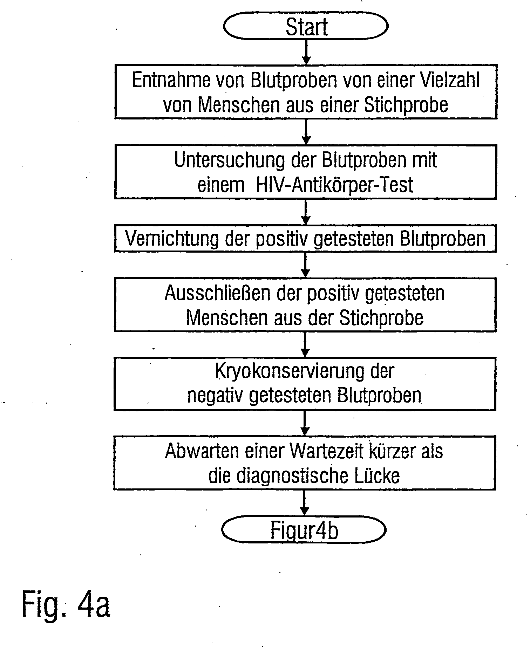

Die Figuren 4a bis 4c zeigen ein alternatives Ausführungsbeispiel eines erfindungsgemäßen Probensammelverfahrens.

Figur 4a zeigt hierbei im Wesentlichen den Abschnitt 1 des Probensammelverfahrens gemäß den Figuren 1a und 1b, so dass diesbezüglich auf die vorangehende Beschreibung verwiesen wird. Der Abschnitt gemäß Figur 4a dient dazu, diejenigen Blutproben auszuschließen, die von Personen stammen, die bereits seit längerem HIV-infiziert sind und deshalb keine "Early-Infection"-Blutprobe liefern können.

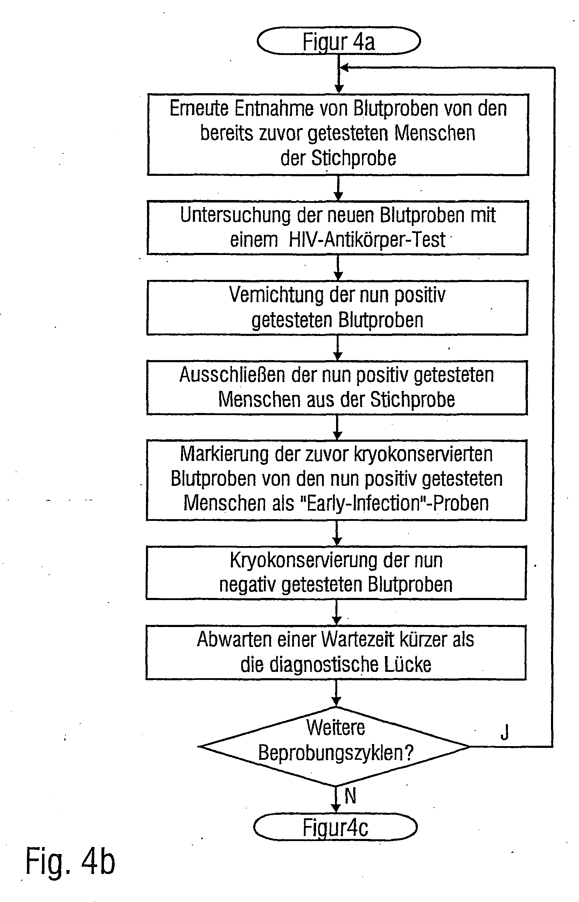

Der Abschnitt in Figur 4b entspricht dagegen im Wesentlichen dem Abschnitt 2 des Ausführungsbeispiels gemäß den Figuren 1a und 1b. Dieser Abschnitt hat die Aufgabe, die Blutproben zu ermitteln, die im Zeitpunkt der ersten Probenentnahme bereits HIV-infiziert waren. Eine Besonderheit dieses Ausführüngsbeispiels besteht darin, dass eine Vielzahl von Beprobungszyklen hintereinander durchgeführt werden können, um die Ausbeute an "Early-Infection"-Blutproben zu erhöhen.

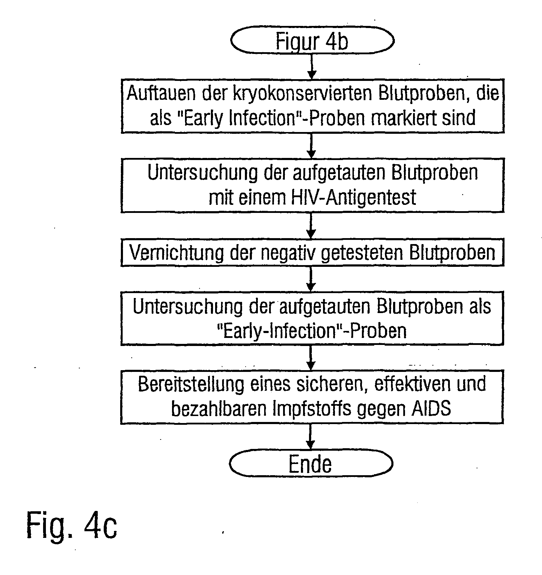

Der Abschnitt gemäß Figur 4c entspricht dann wiederum dem Abschnitt in Figur 1b, so dass diesbezüglich auf die vorstehenden Ausführungsbeispiele verwiesen wird, um Wiederholungen zu vermeiden.

Die Erfindung ist nicht auf die vorstehend beschriebenen bevorzugten Ausführungsbeispiele beschränkt. Vielmehr ist eine Vielzahl von Varianten und Abwandlungen möglich, die ebenfalls von dem Erfindungsgedanken Gebrauch machen und deshalb in den Schutzbereich fallen.

## Patentansprüche

1. Probensammelverfahren zur Gewinnung von Blutproben von frisch HIV-Infizierten in der diagnostischen Lücke eines Antikörper-basierten HIV-Tests, mit den folgenden Schritten:
a) Erste Probeentnahme von biologischen Blutproben von einer Vielzahl von Lebewesen (3-7) einer Stichprobe,
b) Erste Untersuchung der entnommenen Blutproben mit einem ersten Diagnosetest auf eine HIV-virus
c) Ausschließen des zu den positiv getesteten Blutproben gehörenden Lebewesen (3-7) aus der Stichprebe,
d) Kryokonservierung zumindest der negativ getesteten Blutproben,
e) Abwarten einer bestimmten Wartezeit,
f) Zweite Probenentnahme von biologischen Blutproben von den in der Stichprobe verbliebenen Lebewesen (3-7),
g) Zweite Untersuchung der entnommenen Blutproben mit einem zweiten Diagnosetest auf den HIV-Virus,
h) Selektion der kryokonservierten Blutprobe derjenigen Lebewesen (3-7), die zuerst negativ und dann positiv getestet wurden,
i) Auftauen der positiv selektierten kryokonservierten Blutproben,
j) Dritte Untersuchung der aufgetauten Blutproben mit einem dritten Diagnosetest auf den HIV-Virus, wobei der dritte Diagnosetests eine kürzere diagnostische Lücke aufweist als der erste Diagnosetest und der zweite Diagnosetest,
k) Selektion der aufgetauten Blutproben, wobei nur die Blutproben positiv selektiert werden, die bei der dritten Untersuchung positiv getestet werden.

2. Ptobensammelverfahren nach Anspruch 1, **dadurch gekennzeichnet, dass** die Wartezeit zwischen der ersten Probenentnahme und der zweiten Probenentnahme kürzer ist als die zeitliche diagnostische Lücke des zweiten Diagnosetests.

3. Probensammelverzahren nach einem der vorhergehenden Ansprüche, **dadurch gekennzeichnet, dass** der erste Diagnosetest und/oder der zweite Diagnosetest ein Antikörper-basierter Virustest ist, insbesondere ein HIV-Elisa-Test, ist.

4. Probensamrnelvexfahren nach einem der vorhergehenden Ansprüche, **dadurch gekennzeichnet, dass** der dritte Diagnosetest ein Antigen-Test, insbesondere ein HIV-Antigen-Test, ist.

5. Probensammelverfahren nach einem der vorhergehenden Ansprüche, **gekennzeichnet durch** folgenden Schritt: Vernichtung der positiv getesteten Blutproben.

6. Probensammelverfahren nach einem der vorhergehenden Ansprüche, **gekennzeichnet durch** folgenden Schritt: Vernichtung der Blutproben, die bei der dritten Untersuchung negativ getestet wurden.

7. Probensammelverfahren nach einem der vorhergehenden Ansprüche, **dadurch gekennzeichnet, dass** die Kryokonservierung der Blutproben innerhalb einer vorgegebenen Konservierungsfrist nach der jeweiligen Probenentnahme erfolgt.

8. Probensammelverfahren nach Anspruch 7, **dadurch gekennzeichnet, dass** die Konservierungsfrist kürzer als 1 Stunde, 30 Minuten, 10 Minuten oder 5 Minuten ist.

9. Probensammelverfahren nach einem der vorhergehenden Ansprüche, **dadurch gekennzeichnet, dass** die Probenentnahme jeweils dezentral am Aufenthaltsort der zu beprobenden Lebewesen (3-7) erfolgt.

10. Probensammelverfahren nach einem der vorhergehenden Ansprüche, **dadurch gekennzeichnet, dass** die beprobten Lebewesen (3-7) Menschen oder Tiere sind.

## Claims

1. A sample collection method for obtaining blood samples from subjects recently infected with HIV within the diagnostic gap of an antibody-based HIV test, comprising the following steps:
a) first sample taking of biological blood samples from a plurality of creatures (3-7) of a random sample,
b) first examination of the taken blood samples for a HIV virus by means of a first diagnostic test,
c) exclusion of the creatures (3-7) of the random sample associated with the positively tested blood samples,
d) cryoconservation of at least the negatively tested blood samples,
e) waiting for a certain waiting time,
f) second sample taking of biological blood samples from the creatures (3-7) remaining in the random sample,
g) second examination of the taken blood samples for the HIV virus by means of a second diagnostic test,
h) selection of the cryoconserved blood samples of those creatures (3-7) which were initially negatively tested and then positively tested,
i) thawing of the positively selected cryoconserved blood samples,
j) third examination of the thawed blood samples for the HIV virus by means of a third diagnostic test, the third diagnostic test comprising a shorter diagnostic gap than the first diagnostic test and the second diagnostic test,
k) selection of the thawed blood samples, wherein only those blood samples are positively selected, which are positively tested in the third examination.

2. The sample collection method according to claim 1, **characterized in that** the waiting time between the first sample taking and the second sample taking is shorter than the diagnostic gap of the second diagnostic test.

3. The sample collection method according to any one of the preceding claims, **characterized in that** the first diagnostic test and/or the second diagnostic test is an antibody-based virus test, in particular a HIV ELISA test.

4. The sample collection method according to any one of the preceding claims, **characterized in that** the third diagnostic test is an antigen test, in particular a HIV antigen test.

5. The sample collection method according to any one of the preceding claims, **characterized by** the following step:
destruction of the positively tested blood samples.

6. The sample collection method according to any one of the preceding claims, **characterized by** the following step: destruction of the blood samples, which were negatively tested in the third examination.

7. The sample collection method according to any one of the preceding claims, **characterized in that** the cryoconservation of the blood samples takes place within a predetermined conservation period following the respective sample taking.

8. The sample collection method according to claim 7, **characterized in that** the conservation period is shorter than 1 hour, 30 minutes, 10 minutes or 5 minutes.

9. The sample collection method according to any one of the preceding claims, **characterized in that** the sample taking takes place respectively in a decentralized manner at the location of the creatures (3-7) to be sampled.

10. The sample collection method according to any one of the preceding claims, **characterized in that** the sampled creatures (3-7) are humans or animals.

## Revendications

1. Procédé de prélèvement d'échantillons pour obtenir des échantillons de sang parmi des échantillons récemment infectés par le VIH pendant le délai de séroconversion d'un test du VIH basé sur des anticorps, ledit procédé comportant les étapes suivantes :
a) premier prélèvement d'échantillons de sang biologiques sur une pluralité d'organismes (3-7) d'un échantillon aléatoire,
b) premier examen des échantillons de sang prélevés, au moyen d'un premier test diagnostic pour déceler un virus VIH,
c) exclusion des organismes (3-7) faisant partie des échantillons de sang testés positifs, hors de l'échantillon aléatoire,
d) cryoconservation au moins des échantillons de sang testés négatifs,
e) écoulement d'un temps d'attente déterminé,
f) deuxième prélèvement d'échantillons de sang biologiques sur les organismes (3-7) maintenus dans l'échantillon aléatoire,
g) deuxième examen des échantillons de sang prélevés, au moyen d'un deuxième test diagnostic pour déceler le virus VIH,
h) sélection des échantillons de sang, conservés par cryogénie, des organismes (3-7) testés d'abord négatifs et ensuite positifs,
i) décongélation des échantillons de sang conservés par cryogénie et sélectionnés positifs,
j) troisième examen des échantillons de sang décongelés, au moyen d'un troisième test diagnostic pour déceler le virus VIH, le troisième test diagnostic comportant un délai de séroconversion plus court que celui du premier test diagnostic et du deuxième test diagnostic,
k) sélection des échantillons de sang décongelés, seuls les échantillons de sang testés positifs au cours du troisième examen étant sélectionnés comme positifs.

2. Procédé de prélèvement d'échantillons selon la revendication 1, **caractérisé en ce que** le temps d'attente entre le premier prélèvement d'échantillons et le deuxième prélèvement d'échantillons est plus court que le délai de séroconversion du deuxième test diagnostic.

3. Procédé de prélèvement d'échantillons selon l'une quelconque des revendications précédentes, **caractérisé en ce que** le premier test diagnostic et/ou le deuxième test diagnostic est un test du virus basé sur des anticorps, en particulier un test du VIH de type Elisa.

4. Procédé de prélèvement d'échantillons selon l'une quelconque des revendications précédentes, **caractérisé en ce que** le troisième test diagnostic est un test d'antigènes, en particulier un test d'antigènes du VIH.

5. Procédé de prélèvement d'échantillons selon l'une quelconque des revendications précédentes, **caractérisé par** l'étape suivante : destruction des échantillons de sang testés positifs.

6. Procédé de prélèvement d'échantillons selon l'une quelconque des revendications précédentes, **caractérisé par** l'étape suivante : destruction des échantillons de sang qui ont été testés négatifs au cours du troisième examen.

7. Procédé de prélèvement d'échantillons selon l'une quelconque des revendications précédentes, **caractérisé en ce que** la cryoconservation des échantillons de sang est effectuée pendant un délai de conservation prédéfini après le prélèvement d'échantillon respectif.

8. Procédé de prélèvement d'échantillons selon la revendication 7, **caractérisé en ce que** le délai de conservation est inférieur à 1 heure, 30 minutes, 10 minutes ou 5 minutes.

9. Procédé de prélèvement d'échantillons selon l'une quelconque des revendications précédentes, **caractérisé en ce que** le prélèvement d'échantillons est effectué dans chaque cas de manière décentralisée sur le lieu de séjour des organismes (3-7) à tester.

10. Procédé de prélèvement d'échantillons selon l'une quelconque des revendications précédentes, **caractérisé en ce que** les organismes (3-7) à tester sont des humains ou des animaux.
